(19) Europäisches Patentamt
European Patent Office
Office européen des brevets

(11) EP 1 005 850 A1

(12) DEMANDE DE BREVET EUROPEEN

(43) Date de publication:
07.06.2000 Bulletin 2000/23

(51) Int Cl.⁷: **A61K 7/00**

(21) Numéro de dépôt: 99402822.3

(22) Date de dépôt: 15.11.1999

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: 03.12.1998 FR 9815569

(71) Demandeur: **L'OREAL**
**75008 Paris (FR)**

(72) Inventeurs:
• **Roulier, Véronique**
  **75010 Paris (FR)**
• **Cupferman, Sylvie**
  **94240 l'Hay-Les-Roses (FR)**

(74) Mandataire: **Rasson, Catherine**
  **L'OREAL-DPI**
  **6 rue Bertrand Sincholle**
  **92585 Clichy Cedex (FR)**

(54) **Emulsion eau-dans-huile sans conservateur, son procédé de fabrication et son utilisation dans les domaines cosmétique et dermatologique**

(57) L'invention se rapporte à une émulsion comportant une phase aqueuse dispersée dans une phase huileuse, caractérisée en ce qu'elle est exempte de conservateur et en ce que les globules de la phase aqueuse ont une taille moyenne égale ou inférieure à 0,22 microns.

L'invention se rapporte aussi à l'utilisation de ladite émulsion dans les domaines cosmétique et/ou dermatologique, notamment pour le traitement, la protection, le soin et/ou le nettoyage de la peau, des muqueuses et/ou les cheveux, et/ou pour le maquillage de la peau et/ou des muqueuses.

L'invention se rapporte également à un procédé de préparation d'une émulsion eau-dans-huile comportant une phase aqueuse dispersée dans une phase huileuse, les globules de la phase aqueuse ayant une taille moyenne inférieure à 0,22 microns, consistant à introduire sous pression la phase aqueuse dans la phase huileuse, à travers une membrane microporeuse ayant une taille moyenne des pores allant de 0,01 à 0,3 μm à une pression proche de la pression critique.

EP 1 005 850 A1

## Description

L'invention se rapporte à une émulsion eau-dans-huile (E/H) exempte de conservateur, comportant des globules d'eau ayant une taille moyenne inférieure à 0,22 microns, ainsi qu'à un procédé de préparation d'une telle émulsion et à ses utilisations dans les domaines cosmétique et/ou dermatologique.

Dans le domaine cosmétique, il est courant d'utiliser des crèmes constituées d'une émulsion eau-dans-huile (E/H) comportant une phase aqueuse dispersée dans une phase huileuse. Ces émulsions comportent une phase continue huileuse et permettent donc de former à la surface de la peau un film lipidique qui prévient la perte d'eau transépidermique et protège la peau des agressions extérieures. Ces émulsions sont particulièrement appropriées pour protéger et nourrir la peau, et en particulier pour traiter les peaux sèches.

Par ailleurs, Il est courant d'introduire dans les compositions cosmétiques ou dermatologiques, des conservateurs chimiques destinés à lutter contre le développement des micro-organismes dans ces compositions, qui les rendraient rapidement inaptes à l'utilisation. Il faut protéger les compositions à la fois contre les micro-organismes susceptibles de se développer à l'intérieur de la composition et contre ceux que l'utilisateur peut introduire dans celle-ci en la manipulant, et en particulier lors de la préhension avec les doigts de produits en pot.

Parmi les conservateurs chimiques couramment utilisés, on peut citer notamment les parabens ou les donneurs de formol. Ces conservateurs présentent l'inconvénient de causer des intolérances telles que irritations et/ou allergies, et en particulier sur les peaux sensibles. Il en est de même pour les alcools ou les polyols, tels que l'éthanol ou le propylène glycol, en particulier lorsqu'ils sont présents à des taux relativement importants, c'est-à-dire en des quantités excédant 20 % en poids par rapport à l'ensemble de la composition.

En outre, il est connu qu'il est difficile d'obtenir une protection bactériologique efficace pour les émulsions E/H à l'aide des conservateurs, ceux-ci ayant tendance à déstabiliser les émulsions.

La demanderesse a découvert, de manière inattendue, que l'on pouvait préparer des émulsions exemptes de conservateur tout en étant bien protégées contre le développement des micro-organismes, en ayant des globules de phase aqueuse ayant une taille moyenne en nombre et en volume, inférieure à 0,22 microns.

La présente invention a donc pour objet une émulsion comportant une phase aqueuse dispersée dans une phase huileuse, caractérisée en ce qu'elle est exempte de conservateur et en ce que les globules de la phase aqueuse ont une taille moyenne inférieure à 0,22 microns.

L'émulsion de l'invention a l'avantage d'être bien protégée des micro-organismes tout en étant totalement exempte de conservateur et donc non irritante pour les peaux particulièrement sensibles.

La taille moyenne des globules de la phase aqueuse, mesurée en nombre par une méthode de diffraction laser, est inférieure à 0,22 microns, et elle va de préférence de 0,05 à 0,22 microns. Du fait de la finesse de ces globules de phase aqueuse, l'émulsion obtenue présente des qualités sensorielles et visuelles particulièrement satisfaisantes.

La nature de la phase huileuse de l'émulsion selon l'invention n'est pas critique. La phase huileuse peut ainsi être constituée par tous les corps gras et notamment les huiles, classiquement utilisés dans les domaines cosmétique et dermatologique.

Parmi les huiles utilisables dans l'émulsion de l'invention, on peut notamment citer par exemple les huiles végétales (jojoba, avocat), les huiles minérales (isohexadécane, paraffine, isoparaffine, vaseline), les huiles de synthèse (palmitate d'éthylhexyle, myristate d'isopropyle), les huiles de silicone volatiles (cyclométhicones telles que cyclopentadiméthylsiloxane ou cyclohexadiméthylsiloxane) ou non volatiles et les huiles fluorées. Les autres corps gras susceptibles d'être présents dans la phase huileuse peuvent être par exemple les acides gras, les alcools gras et les cires (cire liquide de jojoba).

La phase huileuse de l'émulsion peut représenter de 10 à 80 % en poids et mieux de 15 à 60 % en poids par rapport au poids total de l'émulsion.

L'émulsion de l'invention contient en outre de préférence un émulsionnant approprié pour une émulsion E/H. Comme émulsionnant, on peut citer notamment les émulsionnants siliconés comme les diméthicone copolyols ou les alkyl-$C_{10}$-$C_{22}$-diméthicone copolyols, "alkyl-$C_{10}$-$C_{22}$" signifiant que la chaîne alkyle comporte de 10 à 22 atomes de carbone. Comme émulsionnants, on peut citer par exemple le mélange polyglycéryl-4 isostéarate/ cétyldiméthicone copolyol/ hexyl laurate, commercialisé sous la dénomination Abil WE 09® par la société Goldschmidt, le cétyl diméthicone copolyol tel que le produit commercialisé sous la dénomination Abil EM-90® par la Société Goldschmidt, ou le lauryl diméthicone copolyol comme le produit commercialisé sous la dénomination Q2-5200® de Dow Corning, et un mélange de ces émulsionnants.

La quantité d'émulsionnant peut aller par exemple de 0,1 % à 10 % et de préférence de 0,5 % à 5 % en poids par rapport au poids total de l'émulsion.

L'émulsion peut contenir en outre un co-émulsionnant choisi par exemple parmi les esters d'acide gras et de polyol, les éthers d'alcool gras et de polyol, les éthers d'alcool gras oxyéthylénés et leurs mélanges. Dans ces composés, la chaîne grasse est une chaîne alkyle comportant généralement de 10 à 22 atomes de carbone. Comme co-émulsionnant, on peut citer par exemple l'isostéarate de polyglycéryle-4 tel que le produit vendu par la société Goldschmidt sous la dénomination Isolan GI 34®, l'isostéarate de sorbitanne tel que le produit vendu par la société ICI sous la dénomination Arlacel 987®, l'isostéarate de sorbitanne et de glycérol, tel que le produit vendu par la société ICI sous la dénomination Arlacel 986®, le sesquistéarate de méthylglucose tel que le produit vendu par la société Amerchol sous la dénomination le Glycate IS®.

Quand le co-émulsionnant est présent, la quantité de co-émulsionnant dans l'émulsion de l'invention va avantageusement de 0,1 à 3 % en poids par rapport au poids total de l'émulsion.

L'émulsion selon l'invention peut être utilisée dans tous les domaines où ce type de forme galénique est intéressant, et notamment dans les domaines cosmétique et dermatologique. Quand elle constitue une composition cosmétique et/ou dermatologique, elle contient avantageusement un milieu physiologiquement acceptable, c'est-à-dire compatible avec la peau, les muqueuses, les ongles et/ou les cheveux.

Les émulsions, objet de l'invention, trouvent leur application dans un grand nombre de traitements cosmétiques et/ou dermatologiques de la peau, des muqueuses (lèvres) et des cheveux, y compris le cuir chevelu, notamment pour la protection, le soin, le nettoyage et/ou le maquillage de la peau et/ou des muqueuses, pour la protection, le soin et/ou le nettoyage des cheveux et/ou pour le traitement thérapeutique de la peau, des cheveux et/ou des muqueuses.

Les émulsions selon l'invention peuvent par exemple être utilisées comme produits de soin et/ou de nettoyage pour le visage sous forme de crèmes ou de laits ou comme produits de maquillage (peau et lèvres) par incorporation de charges et/ou de matières colorantes (pigments et/ou colorants).

Aussi, l'invention a encore pour objet l'utilisation cosmétique de l'émulsion telle que définie ci-dessus pour le traitement, la protection, le soin et/ou le nettoyage de la peau, des muqueuses et/ou les cheveux, et/ou pour le maquillage de la peau et/ou des muqueuses.

Les émulsions selon l'invention peuvent être utilisées aussi pour le traitement et la protection dermatologique des peaux sèches.

L'invention a donc aussi pour objet l'utilisation de l'émulsion telle que définie ci-dessus pour la fabrication d'une composition dermatologique destinée au traitement et/ou à la protection des peaux sèches.

En outre, de façon connue, les émulsions de l'invention peuvent contenir des adjuvants habituels dans le domaine cosmétique et/ou dermatologique, tels que des actifs hydrophiles ou lipophiles, des antioxydants, des parfums, des solvants, des charges, des filtres, des matières colorantes, des agents basiques ou acides et encore des vésicules lipidiques. Ces adjuvants sont utilisés dans les proportions habituelles dans le domaine cosmétique ou dermatologique, et par exemple de 0,01 à 30 % du poids total de l'émulsion, et ils sont, selon leur nature, introduits dans la phase aqueuse ou dans la phase huileuse de l'émulsion, ou encore dans des vésicules. Ces adjuvants ainsi que leurs concentrations doivent être tels qu'ils ne modifient pas la propriété recherchée pour l'émulsion.

Comme actifs utilisables dans l'émulsion de l'invention, on peut citer par exemple les hydratants tels que les polyols et notamment la glycérine, l'éthylène glycol, l'isoprène-glycol, le propane-1,2 diol, la diglycérine, le sorbitol, les polyéthylène glycols et leurs mélanges.

Les émulsions de l'invention sont de préférence exemptes de solvant. Ceci va aussi dans le sens d'une émulsion peu agressive et non irritante susceptible d'être utilisée par des personnes à peau sensible. Toutefois, si nécessaire, elles peuvent contenir de faibles quantités d'un solvant, notamment d'un alcool inférieur comportant de un à six atomes de carbone, plus particulièrement l'éthanol. La quantité d'un tel solvant peut aller jusqu'à 5 % en poids par rapport au poids total de l'émulsion.

Les émulsions selon l'invention peuvent être préparées par tout moyen approprié permettant d'obtenir des tailles de globules huileux, inférieures à 0,22 microns. Selon un mode préféré de réalisation de l'invention, elles sont préparées en utilisant une membrane microporeuse, généralement hydrophile, cette technique permettant d'obtenir une taille de globules particulièrement adaptée au but de l'invention. Une telle technique est décrite par exemple dans le document EP-A-546174. La membrane peut être par exemple en verre, en carbone ou en céramique.

Aussi, l'invention a encore pour objet un procédé de préparation d'une émulsion eau-dans-huile comportant une phase aqueuse dispersée dans une phase huileuse, les globules de la phase aqueuse ayant une taille moyenne inférieure à 0,22 microns, consistant à introduire sous pression la phase aqueuse dans la phase huileuse, à travers une membrane microporeuse ayant une taille moyenne des pores allant de 0,01 à 0,3 $\mu m$ et de préférence de 0,05 à 0,2 $\mu m$, à une pression proche de la pression critique.

De préférence, la membrane est préalablement traitée sous vide et aux ultrasons dans de l'eau déminéralisée contenant environ 2 grammes par litre de phase aqueuse de la composition selon l'invention, ce traitement durant environ une heure.

On entend par "pression critique" la pression minimum nécessaire pour l'introduction d'une phase dispersée dans une phase continue à travers une membrane de verre poreuse ayant une taille de pores déterminée. La pression critique (en kPa) est définie par l'équation suivante :

$$Pc = 4\gamma_{ow}\cos\theta/D_m,$$

dans laquelle $\gamma_{ow}$ est la tension interfaciale (mN/m), 0 est l'angle de contact (rad) et $D_m$ est la taille moyenne des pores ($\mu m$) de la membrane.

Dans le procédé de l'invention, la pression utilisée est de préférence proche de la pression critique, c'est-à-dire de Pc + (5 à 20) kPA (0,05 à 0,2 bars).

La pression critique dépend de deux paramètres : la taille des pores de la membrane et l'émulsion à réaliser. L'homme du métier est à même de déterminer la pression critique au cas par cas avant la réalisation de l'émulsion. De manière générale, la pression critique va de 1 kPa à 1000 kPa (0,01 bars à 10 bars).

L'exemple suivant illustre l'invention. Dans cet exemple, les pourcentages sont donnés en poids.

## Exemple

*Phase huileuse*

- Abil EM90        1,5 %
- Isostéarate de polyglycéryle-4 (Isolan GI 34)        0,5 %
- Isohexadécane        15,6 %
- Cyclohexadiméthylsiloxane        10 %
- Expancel (charge)        1 %

*Phase aqueuse*

- Glycérine        5 %
- Sulfate de magnésium        0,7 %
- Eau        qsp 100 %

<u>Mode opératoire</u> : Une membrane en verre ayant une taille de pores de 0,19 μm est immergée dans un litre d'eau déminéralisée contenant 2 grammes de phase aqueuse, puis elle est mise sous vide et aux ultrasons pendant une heure.

Après ce traitement de la membrane, la phase huileuse est pompée pour passer à l'intérieur de la membrane. La phase aqueuse est mise sous pression jusqu'à la pression critique de 65 kPa (0,65 bars). On émulsionne ensuite la phase aqueuse dans la phase huileuse sous une pression de70 kPa (0,7 bars).

On obtient une émulsion très fine, très agréable à l'application et non irritante. En outre, cette émulsion est protégée du point de vue bactériologique.

## Revendications

1. Emulsion eau-dans-huile comportant une phase aqueuse dispersée dans une phase huileuse, caractérisée en ce qu'elle est exempte de conservateur et en ce que les globules de la phase aqueuse ont une taille moyenne inférieure à 0,22 microns.

2. Emulsion selon la revendication 1, caractérisée en ce que la taille moyenne des globules va de 0,05 à 0,22 microns.

3. Emulsion selon la revendication 1 ou 2, caractérisée en ce que la phase huileuse représente de 10 à 80 % en poids par rapport au poids total de l'émulsion.

4. Emulsion selon l'une quelconque des revendications précédentes, caractérisée en ce qu'elle contient en outre au moins un émulsionnant.

5. Emulsion selon la revendication précédente, caractérisée en ce que l'émulsionnant est choisi parmi les diméthicone copolyols et les alkyl-$C_{10}$-$C_{22}$-diméthicone copolyols.

6. Emulsion selon la revendication 4 ou 5, caractérisée en ce que la quantité d'émulsionnant va de 0,1 % à 10 % en poids par rapport au poids total de l'émulsion.

7. Emulsion selon l'une quelconque des revendications précédentes, caractérisée en ce qu'elle contient en outre au moins un co-émulsionnant.

8. Emulsion selon l'une quelconque des revendications précédentes, caractérisée en ce qu'elle constitue une composition cosmétique et/ou dermatologique.

9. Emulsion selon l'une quelconque des revendications précédentes, caractérisée en ce qu'elle contient au moins un additif choisi parmi les actifs hydrophiles, les actifs lipophiles, les antioxydants, les parfums, les solvants, les charges, les filtres solaires, les pigments, les matières colorantes, les agents basiques, les agents acides, les vésicules lipidiques et les gélifiants.

10. Utilisation cosmétique de l'émulsion selon l'une quelconque des revendications 1 à 9 pour le traitement, la protection, le soin et/ou le nettoyage de la peau, des muqueuses et/ou les cheveux, et/ou pour le maquillage de la peau et/ou des muqueuses.

11. Utilisation de l'émulsion selon l'une quelconque des revendications 1 à 9 pour la fabrication d'une composition dermatologique destinée au traitement et/ou à la protection des peaux sèches.

12. Procédé de préparation d'une émulsion eau-dans-huile comportant une phase aqueuse dispersée dans une phase huileuse, les globules de la phase aqueuse ayant une taille moyenne inférieure à 0,22 microns, consistant à introduire sous pression la phase aqueuse dans la phase huileuse, à travers une membrane microporeuse ayant une taille moyenne des pores allant de 0,01 à 0,3 μm à une pression proche de la pression critique (Pc).

13. Procédé de préparation selon la revendication précédente, caractérisé en ce que la pression est égale à Pc + (5 à 20) kPa.

**Office européen des brevets**

## RAPPORT DE RECHERCHE EUROPEENNE

Numéro de la demande

EP 99 40 2822

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | EP 0 686 391 A (L'OREAL) 13 décembre 1995 (1995-12-13) * revendications 1-20 * | 1-3,8-10 | A61K7/00 |
| A | EP 0 530 531 A (BENKISER GMBH) 10 mars 1993 (1993-03-10) | | |

DOMAINES TECHNIQUES RECHERCHES (Int.Cl.7)

A61K

Le présent rapport a été établi pour toutes les revendications

| Lieu de la recherche | Date d'achevement de la recherche | Examinateur |
|---|---|---|
| LA HAYE | 9 mars 2000 | Fouquier, J-P |

CATEGORIE DES DOCUMENTS CITES

X : particulièrement pertinent à lui seul
Y : particulièrement pertinent en combinaison avec un autre document de la même catégorie
A : arrière–plan technologique
O : divulgation non–écrite
P : document intercalaire

T : théorie ou principe à la base de l'invention
E : document de brevet antérieur, mais publié à la date de dépôt ou après cette date
D : cité dans la demande
L : cité pour d'autres raisons
 
& : membre de la même famille, document correspondant

EPO FORM 1503 03.82 (P04C02)

6

**ANNEXE AU RAPPORT DE RECHERCHE EUROPEENNE
RELATIF A LA DEMANDE DE BREVET EUROPEEN NO.**

EP 99 40 2822

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

09-03-2000

| Document brevet cité au rapport de recherche | Date de publication | Membre(s) de la famille de brevet(s) | Date de publication |
|---|---|---|---|
| EP 686391 A | 13-12-1995 | FR 2720644 A<br>CA 2151012 A<br>JP 2656226 B<br>JP 8169808 A<br>US 5643555 A | 08-12-1995<br>07-12-1995<br>24-09-1997<br>02-07-1996<br>01-07-1997 |
| EP 530531 A | 10-03-1993 | DE 4126969 A<br>AT 173391 T<br>DE 59209563 D<br>ES 2124715 T | 18-02-1993<br>15-12-1998<br>24-12-1998<br>16-02-1999 |

EPO FORM P0460